# EUROPÄISCHE PATENTSCHRIFT

(11) **EP 1 626 816 B1**
(45) Veröffentlichungstag und Bekanntmachung des Hinweises auf die Patenterteilung: **09.09.2009**
(21) Anmeldenummer: 04731915.7
(22) Anmeldetag: 10.05.2004
(51) Int. Cl.: B05D 7/24, C23C 18/12, C09D 201/00

(54) **VERFAHREN ZUR BESCHICHTUNG VON SUBSTRATEN MIT KOHLENSTOFFBASIERTEM MATERIAL**
METHOD FOR COATING SUBSTRATES WITH A CARBON-BASED MATERIAL
PROCEDE POUR REVETIR DES SUBSTRATS AVEC UN MATERIAU A BASE DE CARBONE

(30) Priorität: 16.05.2003 DE 10322182; 28.05.2003 DE 10324415
(43) Veröffentlichungstag der Anmeldung: 22.02.2006
(60) Teilanmeldung: 08165697.7
(73) Patentinhaber: CINVENTION AG, 65203 Wiesbaden (DE)
(72) Erfinder: RATHENOW, Jörg, 49610 Quakenbrück (DE); KUNSTMANN, Jürgen, 65812 Bad Soden (DE); MAYER, Bernhard, 63071 Offenbach am Main (DE); BAN, Andreas, 64285 Darmstadt (DE); ASGARI, Soheil, 65203 Wiesbaden (DE)
(74) Vertreter: Hansen, Norbert
(86) Internationale Anmeldenummer: PCT/EP2004/004987
(87) Internationale Veröffentlichungsnummer: WO 2004/101177

(56) Entgegenhaltungen:
- WO-A-97/43473
- WO-A-99/52838
- DE-A- 3 902 856
- DE-A- 10 051 910
- GB-A- 1 163 442
- GB-A- 1 513 235
- US-A- 5 209 979
- US-B1- 6 497 729
- PATENT ABSTRACTS OF JAPAN Bd. 0122, Nr. 70 (C-515), 27. Juli 1988 (1988-07-27) & JP 63 050480 A (DENKI KAGAKU KOGYO KK), 3. März 1988 (1988-03-03)

## Beschreibung

Die vorliegende Erfindung betrifft ein Verfahren zur Beschichtung von Substraten mit kohlenstoffbasiertem Material durch zumindest partielles Beschichten eines Substrates mit einem Polymerfilm an mindestens einer seiner äußeren Oberflächen und anschließende Karbonisierung des Polymerfilms in einer Inertgas-Atmosphäre, bei Temperaturen im Bereich von 200°C bis 2500°C.

Pyrolytischer Kohlenstoff ist seit langem als hochfestes, abriebbeständiges Material mit großer Eigenschaftsvariabilität bekannt. Pyrolytischer Kohlenstoff ist aufgrund seiner Struktur und Zusammensetzungen biokompatibel, so dass er seit langem als Werkstoff oder Beschichtungsmaterial in der Medizintechnik eingesetzt wird, insbesondere zur Herstellung von medizinischen Körperimplantaten aller Art. Pyrolytischer Kohlenstoff mit turbostratischer Struktur, gegebenenfalls unter Einschluss siliziumlegierter Kohlenstoffmikrokristalle wird beispielsweise zur Beschichtung von Stents oder auch zur Herstellung künstlicher Herzklappen verwendet. So beschreibt beispielsweise das US-Patent 6,569,107 kohlenstoffbeschichtete intraluminale Stents, in welchen das Kohlenstoffmaterial mittels chemischer oder physikalischer Dampfphasenabscheidungsmethoden (CVD oder PVD) aufgebracht wurde. In der DE 3902856 werden Pyro-Kohlenstoff enthaltende Formkörper beschrieben, die durch Verkokung von Kohlefasergegenständen, Pyro-Kohlenstoff-Infiltration und anschließende Versiegelung der Oberfläche mit CVD-Kohlenstoff hergestellt werden.
DE 100 51 910 A1 beschreibt die Herstellung von Membranen aus einer flächigen, papierartigen Grundmatrix aus pyrolysierbaren Fasern, wobei diese Grundmatrix zunächst unter Pyrolysebedingungen behandelt wird und anschließend entweder Keramik- oder Kohlenstoff-Vorläufer durch CVD auf der Grundmatrix abgeschieden werden.
GB 1,513,235 betrifft eine Prothese aus kohlenstofffaserverstärktem Kohlenstoff, die verschiedene Beschichtungen, u.a. aus Aluminiumoxid. PTFA oder Keramik versehen sein kann.
US 3,526,005 schließlich betrifft die CVD-Beschichtung einer Prothese mit pyrolytischem Kohlenstoff.

Die Abscheidung von pyrolytischem Kohlenstoff unter PVD- oder CVD-Bedingungen erfordert die sorgfältige Auswahl geeigneter gasförmiger oder verdampfbarer Kohlenstoffprecursoren, die bei hohen Temperaturen zum Teil unter Plasmabedingungen in einer Inertgas- oder Hochvakuumatmosphäre auf ein Substrat abgeschieden werden. Darüber hinaus werden verschiedene Sputterverfahren im

Hochvakuum zur Herstellung von pyrolytischem Kohlenstoff verschiedener Struktur im Stand der Technik beschrieben, siehe beispielsweise US 6,355,350.

Allen diesen Verfahren des Standes der Technik ist gemeinsam, dass die Abscheidung von Kohlenstoffsubstraten unter extremen Temperaturen und/oder Druckbedingungen bei sorgfältiger und aufwendiger Prozesssteuerung stattfindet.

Ferner wird aufgrund unterschiedlicher thermischer Ausdehnungskoeffizienten von Substratmaterial und der aufgebrachten CVD-Kohlenstoffschicht im Stand der Technik oft nur eine geringe Haftung der Schicht auf dem Substrat erzielt, es kommt zu Abplatzungen, Rissen und allgemeinen Verschlechterungen der Oberflächenqualität.

Es besteht daher ein Bedarf nach einfach anwendbaren und kostengünstigen Verfahren zur Beschichtung von Substraten mit kohlenstoffbasiertem Material, welche in der Lage sind, beispielsweise biokompatible oberflächliche Beschichtungen aus Kohlenstoffmaterial oder kohlenstoffbeschichtete Substrate für mikroelektronische Zwecke zur Verfügung zu stellen.

Die Aufgabe der vorliegenden Erfindung ist es daher, ein Verfahren zur Beschichtung von Substraten mit kohlenstoffbasiertem Material zur Verfügung zu stellen, das mit kostengünstigen und vielfältig variierbaren Ausgangsmaterialien auskommt und einfach steuerbare Verarbeitungsbedingungen anwendet.

Eine weitere Aufgabe der vorliegenden Erfindung ist es, mit kohlenstoffbasiertem Material beschichtete Substrate zur Anwendung in der Medizintechnik, insbesondere für medizinische Implantate verschiedener Art zur Verfügung zu stellen, deren Oberflächeneigenschaften sich dem jeweiligen Anwendungszweck entsprechend gezielt anpassen lassen.

Eine weitere Aufgabe der vorliegenden Erfindung liegt in der Bereitstellung eines Verfahrens, zur Herstellung kohlenstoffbeschichteter Substrate für mikroelektronische Zwecke.

Die erfindungsgemäße Lösung der oben genannten Aufgaben besteht in einem Verfahren gemäß Anspruch 1. Bevorzugte Ausführungsformen des erfindungsgemäßen Verfahrens ergeben sich aus den abhängigen Unteransprüchen.

Im Rahmen der vorliegenden Erfindung wurde gefunden, dass sich kohlenstoffbeschichtete Produkte auf einfache Weise dadurch herstellen lassen, dass zunächst ein vorliegendes Substrat oberflächlich zumindest partiell mit einem Polymerfilm beschichtet wird, der anschließend in einer lnertgas-Atmosphäre bei hohen Temperaturen karbonisiert oder pyrolysiert wird.

Unter Karbonisierung oder auch Pyrolyse wird im Rahmen der vorliegenden Erfindung die partielle thermische Zersetzung oder Verkokung kohlenstoffhaltiger Ausgangsverbindungen verstanden, in der Regel Polymermaterialien auf Kohlenwasserstoffbasis, die nach der Karbonisierung hohe Anteile an amorphem Kohlenstoff zurücklassen.

### Substrate

Die erfindungsgemäß verwendbaren Substrate können alle im wesentlichen temperaturbeständigen Materialien umfassen, das heißt Materialien die unter den angewendeten Karbonisierungs- bzw. Pyrolysebedingungen beständig und vorzugsweise formstabil sind. Beispiele für erfindungsgemäß verwendbare Substrate sind Metalle, Legierungen, Glas, Stein, Mineralien, Knochensubstanz und Knochenimitate auf Kalziumkarbonatbasis und dergleichen.

Das erfindungsgemäße Verfahren führt zu mechanisch äussert belastbaren Beschichtungen, und löst insbesondere das Problem von Delaminierungen von herkömmlichen beschichteten Substraten, welche unter harten mechanischen Torsions- Zug- und Dehnungsbelastungen gewöhnlich zu Abrieb von sekundär aufgebrachten Beschichtungen neigen.

Für erfindungsgemäß beschichtete Substrate, insbesondere auch solche für medizinische Zwecke, verwendete Materialien sind alle im medizinischen und zahnmedizinischen Bereich üblicherweise verwendeten Materialien, geeignet zum Beispiel Metalle wie Titan, Platin, Palladium, Gold, Legierungen wie z.B. Kobalt-Chrom-Legierungen Knochensubstanz, Knochenimitate auf Kalziumkarbonatbasis, sowie Kombinationen davon.

Die Substrate können nahezu beliebige äußere Formen aufweisen, sofern sie sich an mindestens einer ihrer äußeren Oberfläche mit einem Polymerfilm beschichten lassen. Bevorzugte Beispiele erfindungsgemäß verwendbarer Substrate sind medizinische Implantate, wie beispielsweise Prothesen und Gelenksubstitute, Knochenimplantate, künstliche Hüftgelenke und Hüftknochenimplantate, intraluminal einsetzbare Vorrichtungen wie Stents, beispielsweise Metallstents wie Nitinolstents, Polymerstents, chirurgisch-orthopädische Hilfsmittel wie Knochenschrauben, Nägel und Platten.

In bevorzugten Ausführungsformen der vorliegenden Erfindung umfassen die zu beschichtenden Substrate Stents, insbesondere Metallstents.

Weitere Beispiele für erfindungsgemäß verwendbare Substrate sind Bauteile aus dem Bereich der Mikroelektronik und Mikromechanik, Konstruktionswerkstoffe wie Metallkeramik in Glas und Stein sowie Raschigringe, Sulzerpackungen, Kartuschen- und Filtersysteme und Isolierwerkstoffe.

### Polymerfilme

Nach dem erfindungsgemäßen Verfahren werden die temperaturbeständigen Substrate an mindestens einer ihrer äußeren Oberflächen zumindest teilweise, in bestimmten bevorzugten Anwendungen, wie beispielsweise bei medizinischen Vorrichtungen in der Regel an ihrer gesamten äußeren Oberfläche mit einem oder mehreren Polymerfilmen beschichtet.

Der Polymerfilm kann in einer Ausführungsform der Erfindung in Form einer Polymerfolie vorliegen, die beispielsweise durch Folienschrumpfverfahren auf das Substrat aufgebracht wird oder auch aufgeklebt werden kann. Thermoplastische Polymerfolien lassen sich auf die meisten Substrate auch in erwärmtem Zustand festhaftend aufbringen.

Geeignete Folien bestehen aus Homo- oder Copolymeren von aliphatischen oder aromatischen Polyolefinen wie Polyethylen, Polypropylen, Polybuten, Polyisobuten, Polypenten; Polybutadien. Polyvinyle wie Polyvinylchlorid oder Polyvinylalkohol; Poly(meth)acrylsäure, Polyacrylnitril, Polyamid, Polyester, Polyurethan, Polystyrol, Polytetrafluorethylen, Wachse, Paraffinwachse, Fischer-Tropsch-Wachse; Mischungen und Kombinationen dieser Homo- oder Copolymere.

In bevorzugten Ausführungsformen werden Polymerfilme und -überzüge auf Basis geschäumter Polymere wie etwa geschäumte Polyolefine, Phenolschäume, Polystyrolschäume, geschäumtes Polyurethan, Fluoropolymerschäume vorteilhaft verwenden. Diese haben den Vorteil, dass sich im Karbonisierungsschritt Beschichtungen mit in Abhängigkeit von der Schaumporosität einstellbarer Porenstruktur erzielen lassen. Zur Herstellung der geschäumten Polymere können alle üblichen Schäumungsverfahren des Standes der Technik unter Verwendung üblicher Treibmittel wie Halogenkohlenwasserstoffe und niedrig siedende Kohlenwasserstoffe verwendet werden.

Gemäß einer weiteren Ausführungsform der vorliegenden Erfindung kann der Polymerfilm auch eine Beschichtung des Substrates umfassen, die ausgewählt ist aus Lacken, Laminaten oder Überzügen. Bevorzugte Überzüge lassen sich durch oberflächliche Parylenierung der Substrate gewinnen. Hierbei werden die Substrate zunächst bei erhöhter Temperatur, üblicherweise etwa 600 °C mit Paracyclophan behandelt, wobei auf den Substraten oberflächlich ein Polymerfilm aus Poly-(p-xylylen) ausgebildet wird. Dieser läßt sich in einem nachfolgenden Karbonisierungs- bzw. Pyrolyseschritt in Kohlenstoff umwandeln.

In bevorzugten Ausführungsformen wird die Schrittfolge Parylenierung und Karbonisierung mehrfach wiederholt.

Geeignete lackbasierte Polymerfilme können beispielsweise aus einem Lack hergestellt werden, der eine Bindemittelbasis aus Alkydharz, Chlorkautschuk, Epoxidharz, Acrylatharz, Phenolharz, Aminharz, Ölbasis, Nitrobasis, Polyester, Polyurethan, Teer, teerartige Materialien, Teerpech, Bitumen, Stärke, Zellulose, Schellack, organische Materialien aus nachwachsenden Rohstoffen oder Kombinationen davon aufweist.

Im erfindungsgemäßen Verfahren können mehrere Schichten aus den genannten Polymerfilmen auf das Implantat aufgebracht werden, welche dann gemeinsam karbonisiert werden. Durch Verwendung unterschiedlicher Polymerfilmmaterialien, evtl. Zusatzstoffe in einzelnen Polymerfilmen oder verschieden dicker Filme können so gezielt Gradientenbeschichtungen auf das Implantat aufgebracht werden, beispielsweise mit veränderlichen Porositäts- oder Adsorptionsprofilen innerhalb der Beschichtungen. Ferner kann die Schrittfolge Polymerfilmbeschichtung und Karbonisierung einmal und ggf. auch mehrfach wiederholt werden um kohlenstoffhaltige Multilayerbeschichtungen auf dem Implantat zu erhalten. Hierbei können die Polymerfilme oder Substrate vorstrukturiert oder mittels Zusatzstoffen modifiziert werden. Auch geeignete Nachbehandlungsschritte wie im weiteren beschrieben können nach jedem oder nach einzelnen Schrittfolgen Polymerfilmbeschichtung und Karbonisierung des erfindungsgemäßen Verfahrens angewendet werden, wie beispielsweise eine oxidative Behandlung einzelner Schichten.

Auch die Verwendung mittels oben genannter Lacke oder Beschichtungslösungen beschichteter Polymerfilme zur Beschichtung der Implantate mittels beispielsweise Kaschiertechniken wie thermisch, Druckgepresst, oder naß-in-naß ist erfindungsgemäß vorteilhaft anwendbar.

In bestimmten Ausführungsformen der vorliegenden Erfindung kann der Polymerfilm mit Zusatzstoffen ausgerüstet sein, welche das Karbonisierungsverhalten des Films und/oder die makroskopischen Eigenschaften der aus dem Verfahren resultierenden kohlenstoffbasierten Substratbeschichtung beeinflusst. Beispiele geeigneter Zusatzstoffe sind Füllstoffe, Porenbildner, Metalle und Metallpulver, etc. Beispiele für anorganische Zusatz -oder Füllstoffe sind Siliziumoxide oder Aluminiumoxide, Aluminosilikate, Zeolithe, Zirkonoxide, Titanoxide, Talkum, Graphit, Ruß, Fullerene, Tonmaterialien, Phyllosilikate, Silicide, Nitride, Metallpulver, insbesondere von katalytisch aktiven Übergangsmetallen wie Kupfer, Gold und Silber, Titan, Zirkon, Hafnium, Vanadium, Niob, Tantal, Chrom, Molybdän, Wolfram, Mangan, Rhenium, Eisen, Kobalt, Nickel, Ruthenium, Rhodium, Palladium, Osmium, Iridium oder Platin.

Mittels derartiger Zusatzstoffe im Polymerfilm lassen sich beispielsweise biologische, mechanische und thermische Eigenschaften der Filme wie auch der resultierenden Kohlenstoffbeschichtungen variieren und einstellen. So kann z.B. durch den Einbau von Schichtsilikaten der thermische Ausdehnungskoeffizient der Kohlenstoffschicht dem eines Substrat aus Keramik angeglichen werden, so dass die aufgebrachte kohlenstoffbasierte Beschichtung auch bei starken Temperaturdifferenzen fest haftet. So wird der Zusatz von Aluminium basierten-Füllstoffen zu einer Erhöhung des thermischen Ausdehnungskoeffizienten und durch den Zusatz von Glas- , Graphit- oder Quartz-basierten Füllstoffen zu einer Verminderung des thermischen Ausdehnungskoeffizienten führen, so dass durch Mischung der Komponenten im Polymersystem der thermische Ausdehnungskoeffizioent entsprechend individuell eingestellt werden kann. Eine weitere mögliche Einstellung der Eigenschaften kann beispielsweise und nicht ausschließlich durch die Herstellung eines Faserverbunds mittels Zusatz von Kohlenstoff-, Polymer-, Glas- oder anderen Fasern in gewebter oder nicht gewebter Form erfolgen, welches zu einer deutlichen Steigerung der Elastizität der Beschichtung führt.

Polymerfilme haben den Vorteil, dass sie sich einfach in nahezu beliebigen Dimensionen herstellen lassen oder kommerziell erhältlich sind. Polymerfolien sind leicht verfügbar, kostengünstig und auf Substrate verschiedenster Art einfach aufzubringen. Die erfindungsgemäß verwendeten Polymerfilme können vor der Pyrolyse bzw. Karbonisierung durch Falten, Prägen, Stanzen, Drucken, Extrudieren, Raffen, Spritzgiessen und dergleichen in geeigneter Weise strukturiert werden, bevor oder nachdem sie auf das Substrat aufgebracht werden. Auf diese Weise lassen sich in die nach dem erfindungsgemäßen Verfahren hergestellte Kohlenstoffbeschichtung bestimmte Strukturen regelmäßiger Art oder unregelmäßiger Art einbauen.

Die erfindungsgemäß verwendbaren Polymerfilme aus Beschichtungen in Form von Lacken oder Überzügen können aus dem flüssigen, breiigen oder pastenförmigen Zustand, zum Beispiel durch Anstreichen, Streichen, Lackieren, Dispersions- oder Schmelzbeschichten, Extrudieren, Gießen, Tauchen oder auch als Hotmelts, aus dem festen Zustand mittels Pulverbeschichtung, Klammspritzverfahren, Sintern oder dergleichen nach an sich bekannten Verfahren auf das Substrat aufgebraucht werden. Auch das Kaschieren von geeignet geformten Substraten mit hierfür geeigneten Polymermaterialien oder Folien ist ein erfindungsgemäß verwendbares Verfahren zur Beschichtung des Substrats mit einem Polymerfilm.

Besonders bevorzugt bei der Beschichtung von Substraten mit Polymerfilmen ist die Auftragung des Polymers bzw. einer Lösung davon mittels Druckverfahren wie in der DE 10311150 beschrieben. Dieses Verfahren ermöglicht insbesondere eine präzise und reproduzierbare Einstellung der Schichtdicke des aufgetragenen Polymermaterials.

### Karbonisierung

Der auf das Substrat aufgebrachte Polymerfilm wird gegebenenfalls getrocknet und anschließend einer pyrolytischen Zersetzung unter Karbonisierungsbedingungen unterzogen. Hierbei wird der auf dem Substrat aufbeschichtete Polymerfilm in einer lnertgas-Atmosphäre bei erhöhter Temperatur karbonisiert. Die Temperatur des Karbonisierungsschritts liegt im Bereich von 200°C bis 2500°C und wird vom Fachmann in Abhängigkeit von den spezifischen temperaturabhängigen Eigenschaften der verwendeten Polymerfilme und Substrate gewählt.

Bevorzugte allgemein verwendbare Temperaturen für den Karbonisierungsschritt des erfindungsgemäßen Verfahrens liegen bei 200°C bis etwa 1200°C. Bei einigen Ausführungsformen sind Temperaturen im Bereich von 250°C bis 700°C bevorzugt. Generell wird die Temperatur je nach den Eigenschaften der verwendeten Materialien so gewählt, dass der Polymerfilm mit möglichst geringem Temperaturaufwand im wesentlichen vollständig zu kohlenstoffhaltigem Feststoff überführt wird. Durch die geeignete Wahl bzw. Steuerung der Pyrolysetemperatur kann die Porosität, die Festigkeit und die Steife des Materials sowie weitere Eigenschaften gezielt eingestellt werden.

Vorzugsweise wird Porosität in den erfindungsgemäßen Schichten auf Implantaten durch Behandlungsverfahren erzeugt, wie sie in der DE 103 35 131 und der PCT/EP04/00077 beschrieben werden.

Durch Anwendung sehr hoher Temperaturen bis zu 2000 °C und mehr lassen sich erfindungsgemäße kohlenstoffbasierte Beschichtungen in graphitischer Form herstellen. Eine geeignete Wahl der Karbonisierungstemperatur erlaubt die gezielte Eistellung der Kristallinität der Beschichtungen von vollständig amorph bei niederen Temperaturen bis zu hochkristallinen bei hohen Temperaturen. Damit lassen sich auch die mechanischen Eigenschaften der Beschichtungen gezielt nach dem jeweiligen Anwendungszweck einstellen und optimieren.

Die Atmosphäre beim Karbonisierungsschritt des erfindungsgemäßen Verfahrens ist im wesentlichen frei von Sauerstoff. Erfindungsgemäß ist die Verwendung von Inertgas-atmosphären, beispielsweise aus Stickstoff, Edelgasen wie Argon, Neon sowie beliebige andere inerte, nicht mit Kohlenstoff reagierende Gase oder Gasverbindungen sowie auch Mischungen von inerten Gasen. Bevorzugt sind Stickstoff und/oder Argon.

Die Karbonisierung wird üblicherweise bei Normaldruck in Gegenwart von inerten Gasen wie den oben genannten durchgeführt. Gegebenenfalls sind jedoch auch höhere Inertgasdrücke vorteilhaft verwendbar. In bestimmten Ausführungsformen des erfindungsgemäßen Verfahrens kann die Karbonisierung auch bei Unterdruck bzw. im Vakuum erfolgen.

### Ofenprozess

Der Pyrolyseschritt wird vorzugsweise in einem kontinuierlichen Ofenprozess durchgeführt. Die ggf. strukturierten, beschichteten oder vorbehandelten Polymerfilme werden dabei auf einer Seite dem Ofen zugeführt und am anderen Ende des Ofens wieder austreten. In bevorzugten Ausführungsformen kann der Polymerfilm bzw. der aus Polymerfilmen geformte Gegenstand im Ofen auf einer Lochplatte, einem Sieb oder dergleichen aufliegen, so dass durch den Polymerfilm während der Pyrolyse und/oder Karbonisierung Unterdruck angelegt werden kann. Dies ermöglicht nicht nur eine einfache Fixierung der Gegenstände im Ofen, sondern auch eine Absaugung und optimale Durchströmung der Filme bzw. Baugruppen mit Inertgas während der Pyrolyse und/oder Karbonisierung.

Der Ofen kann durch entsprechende Inertgasschleusen in einzelne Segmente unterteil werden, in welchen nacheinander ein oder mehrere Pyrolyse- bzw. Karbonisierungsschritte, ggf. bei unterschiedlichen Pyrolyse- bzw. Karbonisierungsbedingungen wie zum Beispiel unterschiedlichen Temperaturstufen, unterschiedlichen Inertgasen bzw. Vakuum durchgeführt werden können.

Ferner können in entsprechenden Segmenten des Ofens ggf. auch Nachbehandlungsschritte wie Nachaktivieren durch Reduktion oder Oxidation oder Imprägnierung mit Metallsalzlösungen etc. durchgeführt werden.

Alternativ hierzu kann die Pyrolyse/Karbonisierung auch in einem geschlossenen Ofen durchgeführt werden, was insbesondere dann bevorzugt ist, wenn die Pyrolyse und/oder Karbonisierung im Vakuum durchgeführt werden soll.

Während der Pyrolyse und/oder Karbonisierung im erfindungsgemäßen Verfahren tritt üblicherweise eine Gewichtsabnahme des Polymerfilms von ca. 5 % bis 95 %, vorzugsweise ca. 40 % bis 90 %, insbesondere 50 % bis 70 %, je nach verwendetem Ausgangsmaterial und Vorbehandlung auf. Darüber hinaus tritt während der Pyrolyse und/oder Karbonisierung im erfindungsgemäßen Verfahren in der Regel ein Schrumpf des Polymerfilms bzw. der aus Polymerfilmen erzeugten Struktur bzw. Baugruppe auf. Der Schrumpf kann in einer Größenordnung von 0 % bis etwa 95 %, vorzugsweise bei 10 % bis 30 % liegen.

Im erfindungsgemäßen Verfahren kann die elektrische Leitfähigkeit der Beschichtung in Abhängigkeit von der verwendeten Pyrolyse- bzw. Karbonisierungstemperatur und der Art und Menge des eingesetzten Zusatzstoffs bzw. Füllmaterials in weiten Berreichen eingestellt werden. Dies ist insbesondere für Anwendungen in der Mikroelektronik vorteilhaft. So kann bei Temperaturen im Bereich von 1000 bis 2500 °C infolge der auftretenden Graphitisierung der Beschichtung eine höhere Leitfähigkeit erreicht werden als bei tieferen Temperaturen. Daneben kann die elektrische Leitfähigkeit aber auch beispielsweise durch Zusatz von Graphit zum Polymerfilm erhöht werden, welcher dann bei niedrigeren Temperaturen pyrolysiert bzw. karbonisiert werden kann. Derartig modifizierte beschichtete Substrate eignen sich beispielsweise für die Herstellung von Sensoren.

Die erfindungsgemäß hergestellte kohlenstoffbasierte Beschichtung weist, je nach Ausgangsmaterial, Menge und Art der Füllmaterialien, einen Kohlenstoffgehalt von mindestens 1 Gew.-% auf, vorzugsweise mindestens 25 %, gegebenenfalls auch mindestens 60 % und insbesondere bevorzugt mindestens 75 %. Erfindungsgemäß besonders bevorzugte Beschichtungen weisen einen Kohlenstoffgehalt von mindestens 50 Gew.-% auf

### Nachbehandlung

In bevorzugten Ausführungsformen des erfindungsgemäßen Verfahrens können die physikalischen und chemischen Eigenschaften der resultierenden kohlenstoffhaltigen Beschichtung des Substrats nach der Karbonisierung durch geeignete Nachbehandlungsschritte weiter modifiziert und dem jeweils gewünschten Verwendungszweck angepasst werden.

Durch ein- oder beidseitige Beschichtung des Polymerfilms mit Epoxydharzen, Phenolharz, Teer, Teerpech, Bitumen, Kautschuk, Polychloropren oder Poly(styrol-co-butadien)-Latexmaterialien, Siloxane, Silikate, Metallsalze bzw.

Metallsalzlösungen, beispielsweise Übergangsmetallsalze, Russ, Fullerene, Aktivkohlepulver, Kohlenstoffmolekularsieb, Perowskit, Aluminiumoxide, Siliziumoxide, Siliziumcarbid, Bornitrid, Siliziumnitrid, Edelmetallpulver wie beispielsweise Pt, Pd, Au oder Ag; sowie Kombinationen davon, oder auch durch gezielten Einbau derartiger Materialien in die Polymerfilmstruktur lassen sich die Eigenschaften des nach der Pyrolyse und/oder Karbonisierung resultierenden porösen kohlenstoffbasierten Beschichtungen gezielt beeinflussen und veredeln, oder auch Multilayer-Beschichtungen herstellen. Beispielsweise kann durch Einbau von Schichtsilikaten in den Polymerfilm oder Beschichtung des Polymerfilms mit Schichtsilikaten, Nanopartikeln, anorganischen Nanokompositen Metallen, Metalloxiden und dergleichen der thermische Ausdehnungskoeffizient der resultierenden Kohlenstoffbeschichtungen wie auch z.B. dessen mechanische Eigenschaften modifiziert werden.

Bei der erfindungsgemäßen Herstellung von beschichteten Substraten, besteht durch den Einbau oben genannter Zusatzstoffe in den Polymerfilm die Möglichkeit, die Haftung der aufgebrachten Schicht auf dem Substrat zu verbessern und beispielsweise den thermischen Ausdehnungskoeffizienten der äußeren Schicht demjenigen des Substrats anzupassen, so dass diese beschichteten Substrate beständiger gegenüber Brüchen in und Abplatzen der Beschichtung werden. Diese Beschichtungen sind somit haltbarer und langzeitstabiler im konkreten Einsatz als herkömmliche Produkte dieser Art.

Die Aufbringung oder der Einbau von Metallen und Metallsalzen, insbesondere auch von Edelmetallen und Übergangsmetallen ermöglicht es, die chemischen, biologischen und adsorptiven Eigenschaften der resultierenden kohlenstoffbasierten Beschichtungen jeweils erwünschten Erfordernissen anzupassen, so dass die resultierende Beschichtung für besondere Anwendungen beispielsweise auch mit heterogenkatalytischen Eigenschaften ausgerüstet werden kann.

In bevorzugten Ausführungsformen des erfindungsgemäßen Verfahrens werden die physikalischen und chemischen Eigenschaften der kohlenstoffbasierten Beschichtung nach der Pyrolyse bzw. Karbonisierung durch geeignete Nachbehandlungsschritte weiter modifiziert und dem jeweils gewünschten Verwendungszweck angepasst.

Geeignete Nachbehandlungen sind beispielsweise reduzierende oder oxidative Nachbehandlungsschritte, bei welchem die Beschichtung mit geeigneten Reduktionsmitteln und/oder Oxidationsmitteln wie Wasserstoff, Kohlendioxid, Wasserdampf, Sauerstoff, Luft, Salpetersäure und dergleichen sowie ggf. Mischungen dieser behandelt wird.

Die Nachbehandlungsschritte können ggf. bei erhöhter Temperatur, jedoch unterhalb der Pyrolysetemperatur, beispielsweise von 40°C bis 1000°C, vorzugsweise 70°C bis 900°C, besonders bevorzugt 100°C bis 850°C, insbesondere bevorzugt 200°C bis 800°C und insbesondere bei etwa 700 °C durchgeführt werden. In besonders bevorzugten Ausführungsformen wird die erfindungsgemäß hergestellte Beschichtung reduktiv oder oxidativ, oder mit einer Kombination dieser Nachbehandlungsschritte bei Raumtemperatur modifiziert.

Durch oxidative bzw. reduktive Behandlung, oder auch den Einbau von Zusatzstoffen, Füllstoffen oder funktionellen Materialien lassen sich die Oberflächeneigenschaften der erfindungsgemäß hergestellten Beschichtungen gezielt beeinflussen bzw. verändern. Beispielsweise können durch Einbau von anorganischen Nanopartikeln oder Nanokompositen wie Schichtsilikaten die Oberflächeneigenschaften der Beschichtung hydrophilisiert oder hydrophobisiert werden.

Ferner können mittels Ionenimplantierung die Oberflächeneigenschaften des beschichteten Substrats modifiziert werden. So können durch Implantierung von Stickstoff Nitrid-, Carbonitrid- oder Oxynitridphasen mit eingelagerten Übergangsmetallen gebildet werden, was die chemische Resistenz und mechanische Widerstandsfähigkeit der kohlenstoffhaltigen Beschichtungen deutlich erhöht. Die Ionenimplantierung von Kohlenstoff kann zur Erhöhung der mechanischen Festigkeit der Beschichtungen wie auch zur Nachverdichtung poröser Schichten verwendet werden.

Auch können die erfindungsgemäß hergestellten Beschichtungen nachträglich durch Einbau geeigneter Zusatzstoffe mit biokompatiblen Oberflächen ausgestattet und gegebenenfalls als Bioreaktoren oder Arzneistoffträger eingesetzt werden. Hierzu können z.B. Medikamente oder Enzyme in das Material eingebracht werden, wobei erstere ggf. durch geeignete Retardierung und/oder selektive Permeationseigenschaften der Beschichtungen kontrolliert freigesetzt werden können.

Ferner ist es in bestimmten Ausführungsformen bevorzugt, die erfindungsgemäß hergestellten Beschichtungen zu fluoridieren, beispielsweise um Oberflächenbeschichtete Stents zur Aufnahme lipophiler Stoffe bzw. Wirkstoffe zu befähigen.

Auch kann nach dem erfindungsgemäßen Verfahren die Beschichtung auf dem Substrat geeignet modifiziert werden, z.B. durch Variation der Porengrößen mittels geeigneter Nachbehandlungsschritte, so dass die kohlenstoffbasierte Beschichtung das Wachstum von Mikroorganismen oder lebenden Zellen begünstigt bzw. fördert. Entsprechend beschichtete Substrate können dann beispielsweise in Bioreaktoren als Wachstumsmedium für Mikroorganismen dienen. Vorteilhafterweise lässt sich die Porosität der Beschichtung so einstellen, dass die Versorgung der auf der äußeren Oberfläche angesiedelten Zellen oder Mikroorganismen mit Nährstoffen durch im oder auf dem Substrat liegende Nährmittel- oder Wirkstoffdepots gewährleistet werden kann, wobei die Nährstoffe aus dem Substrat durch Permeation durch die kohlenstoffbasierte Beschichtung an die oberflächliche Mikroorganismenbesiedlung gelangen.

Die karbonisierte Beschichtung kann gegebenenfalls auch in einem weiteren optionalen Verfahrensschritt, einem sogenannten CVD-Prozeß (Chemical Vapour Deposition, chemische Gasphasenabscheidung) unterzogen werden, um die Oberflächen- oder Porenstruktur und deren Eigenschaften weiter zu modifizieren. Hierzu wird die karbonisierte Beschichtung mit geeigneten Precursorgasen bei hohen Temperaturen behandelt. Derartige Verfahren sind im Stand der Technik seit langem bekannt.

Als Kohlenstoff-abspaltende Precursor kommen nahezu alle bekannten gesättigten und ungesättigten Kohlenwasserstoffe mit ausreichender Flüchtigkeit unter CVD-Bedingungen in Frage. Beispiele hierfür sind Methan, Ethan, Ethylen, Acetylen, lineare und verzweigte Alkane, Alkene und Alkane mit Kohlenstoffzahlen von C₁-C₂₀, aromatische Kohlenwasserstoffe wie Benzol, Naphthalin etc., sowie ein- und mehrfach alkyl-, alkenyl- und alkinylsubstituierte Aromaten wie beispielsweise Toluol, Xylol, Cresol, Styrol.

Als Keramik-Precursor können BCl₃, NH₃, Silane wie Tetraethoxysilan (TEOS), SiH₄, Dichlorodimethylsilan (DDS), Methyltrichlorosilan (MTS), Trichlorosilyldichloroboran (TDADB), Hexadichloromethylsilyloxid (HDMSO), AlCl₃, TiCl₃ oder Mischungen davon verwendet werden.

Diese Precursor werden in CVD-Verfahren zumeist in geringer Konzentration von etwa 0,5 bis 15 Vol.-% in Mischung mit einem Inertgas, wie beispielweise Stickstoff, Argon oder dergleichen angewendet. Auch der Zusatz von Wasserstoff zu entsprechenden Abscheidegasgemischen ist möglich. Bei Temperaturen zwischen 500 und 2000°C, vorzugsweise 500 bis 1500°C und besonders bevorzugt 700 bis 1300°C, spalten die genannten Verbindungen Kohlenwasserstofffragmente bzw. Kohlenstoff oder keramische Vorstufen ab, die sich im Porensystem der pyrolysierten Beschichtung im wesentlichen gleichmäßig verteilt niederschlagen, dort die Porenstruktur modifizieren und so zu einer im wesentlichen homogenen Porengröße und Porenverteilung im Sinne einer weiteren Optimierung führen.

Die erfindungsgemäß hergestellten kohlenstoffhaltigen Beschichtungen weisen aussergewöhnlich gute mechanische Festigkeit auf. Erfindungsgemäße Beschichtungen auf Edelstahl (z.B. 316L) zeigen üblicherweise Elastizitätsmodule von etwa10-30 GPa, Härten nach Vickers von etwa 200 bis 600, typischerweise etwa 400, und Friktionskoeffizienten von etwa 0,03 bis 0,2, typischerweise etwa 0,14. Frakturen in der Schicht werden erst ab etwa 30-60 mN (Scratch Adhesion) beobachtet, Abrasion ab etwa 40 bis 400 mN.

Pyrolytischer Kohlenstoff ist in der Regel ein hoch bioverträgliches Material, das bei medizinischen Anwendungen wie beispielsweise der äußeren Beschichtung von Implantaten verwendet werden kann. Die Biokompatibilität der erfindungsgemäß beschichteten Substrate kann ferner durch den Einbau von Zusatzstoffen, Füllstoffen, Proteinen oder funktionellen Materialien und/oder Medikamente in die Polymerfilme vor der Karbonisierung gezielt beeinflusst, bzw. verändert werden, wie oben erwähnt. Hierdurch lassen sich Abstoßungsphänomene im Körper bei erfmdungsgemäß hergestellten Implantaten verringern oder ganz ausschalten.

In besonders bevorzugten Ausführungsformen können erfindungsgemäß hergestellte kohlenstoffbeschichtete medizinische Implantate durch gezielte Einstellung der Porosität der aufgebrachten Kohlenstoffschicht zur kontrollierten Abgabe von Wirkstoffen aus dem Substrat in die äußere Umgebung verwendet werden. Auf diese Weise lassen sich beispielsweise medizinische Implantate als Arzneistoffträger mit Depotwirkung verwenden, wobei die kohlenstoffbasierte Beschichtung des Implantats als freisetzungsregulierende Membran genutzt werden kann. Auch können auf die bioverträglichen Beschichtungen Arzneistoffe aufgebracht werden. Dies ist insbesondere da nützlich, wo Wirkstoffe nicht im oder auf dem Substrat direkt aufgebracht werden können, wie bei Metallen.

Ferner können die erfindungsgemäß hergestellten Beschichtungen in einem weiteren Verfahrensschritt mit Arzneistoffen bzw. Medikamenten beladen werden, oder auch mit Markern, Kontrastmitteln zur Lokalisierung von beschichteten Implantaten im Körper, oder auch mit therapeutischen oder diagnostischen Mengen an radioaktiven Strahlern. Für letzteres sind die erfindungsgemäßen Beschichtungen auf Kohlenstoffbasis besonders geeignet, da sie im Gegensatz zu Polymerschichten von radioaktiver Strahlung nicht verändert bzw. angegriffen werden.

Im medizinischen Bereich erweisen sich erfindungsgemäß beschichtete Implantate als besonders langzeitstabil, da die kohlenstoffbasierten Beschichtungen neben ihrer hohen Festigkeit auch hinreichend elastisch und flexibel sind, so dass sie den Bewegungen des Implantats, insbesondere bei hochbelasteten Gelenken, folgen können ohne dass die Gefahr besteht, dass sich Risse bilden oder die Schicht abblättert.

Die Erfindung wird nun im Folgenden anhand von Beispielen näher erläutert, die bevorzugte Ausführungsformen darstellen, welche keine notwendigen Beschränkungen der Erfindung wie in den Ansprüchen beschrieben wiedergeben:

### Beispiele

### Beispiel 1: Kohlenstoff (Vergleichsbeispiel)

Ein beschichtetes Kohlenstoffmaterial wurde wie folgt hergestellt: Auf ein Papier mit 38g/m² Flächengewicht als Grünkörper wurde ein Polymerfilm aufgetragen, indem das Papier mehrfach mit einer Rakel mit einem handelsüblichen epoxidierten Phenolharzlack beschichtet und bei Raumtemperatur getrocknet wurde. Trockengewicht 125g/m². Die Pyrolyse bei 800°C über 48 Stunden unter Stickstoff liefert bei einem Schrumpf von 20% und einen Gewichtsverlust von 57% ein asymmetrisch aufgebautes Kohlenstoffblatt mit folgenden Abmessungen: Gesamtstärke 50 Mikrometer, mit 10 Mikrometern einer dichten erfindungsgemäßen kohlenstoffhaltigen Schicht auf einem offenporiger Kohlenstoffträger mit einer Stärke von 40 Mikrometern, der sich unter den Pyrolysebedingungen in situ aus dem Papier bildete. Das Absorptionsvermögen des beschichteten Kohlenstoffmaterials betrug bis zu 18 g Ethanol / m².

### Beispiel 2: Glas

Duroplan® Glas wird einer 15 min. Ultraschallreinigung in einem tensidhaltigen Wasserbad unterworfen, mit destilliertem Wasser und Aceton gespült und getrocknet. Dieses Material wird durch Tauchbeschichtung mit einem handelsüblichen Emballagelack auf Phenolharzbasis mit 2,0*10⁻⁴ g/cm² Auftragsgewicht beschichtet. Nach anschließender Karbonisierung bei 800°C über 48 Stunden unter Stickstoff tritt ein Gewichtsverlust der Beschichtung auf 0,33*10⁻⁴ g/cm² ein. Die zuvor farblose Beschichtung wird schwarz glänzend, und ist nach der Karbonisierung kaum mehr durchsichtig. Ein Test der Beschichtungshärte mit einem Bleistift, der in einem Winkel von 45° mit einem Gewicht von 1kg über die beschichtete Fläche gezogen wird ergibt bis zu einer Härte von 5H keine optisch wahrnehmbare Beschädigung der Oberfläche.

### Beispiel 3: Glas, CVD-Beschichtung (Vergleichsbeispiel)

Duroplan® Glas wird einer 15 min. Ultraschallreinigung unterworfen, mit dest. Wasser und Aceton gespült und getrocknet. Dieses Material wird durch chemische Dampfabscheidung (CVD) mit 0,05*10⁻⁴ g/cm² Kohlenstoff beschichtet. Hierzu wird Benzol bei 30°C in einem Blubberer durch einen Stickstofffluß über 30 Minuten in Kontakt mit der 1000°C heißen Glasoberfläche gebracht und als Film auf der Glasoberfläche abgeschieden. Die zuvor farblose Glasoberfläche wird grau glänzend und ist nach der Abscheidung gedämpft durchsichtig. Ein Test der Beschichtungshärte mit einem Bleistift, der in einem Winkel von 45° mit einem Gewicht von 1kg über die beschichtete Fläche gezogen wird ergibt bis zu einer Härte von 6B keine optisch wahrnehmbare Beschädigung der Oberfläche.

### Beispiel 4: Glasfaser

Duroplan® Glasfasern mit 200 Mikrometern Durchmesser, werden einer 15 min. Ultraschallreinigung unterworfen, mit dest. Wasser und Aceton gespült und getrocknet. Dieses Material wird durch Tauchbeschichtung mit einem handelsüblichen Emballagelack mit 2,0*10⁻⁴ g/cm² Auftragsgewicht beschichtet. Nach anschließender Pyrolyse mit Karbonisierung bei 800°C über 48 Stunden tritt ein Gewichtsverlust der Beschichtung auf 0,33*10⁻⁴ g/cm² ein. Die zuvor farblose Beschichtung wird schwarz glänzend, und ist nach der Karbonisierung kaum mehr durchsichtig. Ein Test der Haftung der Beschichtung durch Biegung im Radius von 180° ergibt keine Abplatzungen, d.h. optisch wahrnehmbare Beschädigung der Oberfläche.

### Beispiel 5: Edelstahl

Edelstahl 1.4301 als 0,1mm Folie (Goodfellow) wird einer 15 min. Ultraschallreinigung unterworfen, mit dest. Wasser und Aceton gespült und getrocknet. Dieses Material wird durch Tauchbeschichtung mit einem handelsüblichen Emballagelack mit 2,0*10⁻⁴ g/cm² Auftragsgewicht beschichtet. Nach anschließender Pyrolyse mit Karbonisierung bei 800°C über 48 Stunden unter Stickstoff tritt ein Gewichtsverlust der Beschichtung auf 0,49*10⁻⁴ g/cm² ein. Die zuvor farblose Beschichtung wird nach der Karbonisierung matt schwarz. Ein Test der Beschichtungshärte mit einem Bleistift, der in einem Winkel von 45° mit einem Gewicht von 1kg über die beschichtete Fläche gezogen wird ergibt bis zu einer Härte von 4B keine optisch wahrnehmbare Beschädigung der Oberfläche. Ein Klebestreifen-Abziehtest, bei dem ein Tesa®-Streifen mit mindestens 3 cm Länge über 60 Sekunden mit dem Daumen auf die Oberfläche geklebt wird und anschließend im Winkel von 90° von der Oberfläche wieder abgezogen wird, ergibt kaum Anhaftungen.

### Beispiel 6: Edelstahl, CVD-Beschichtung (Vergleichsbeispiel)

Edelstahl 1.4301 als 0,1mm Folie (Goodfellow) wird einer 15 min. Ultraschallreinigung unterworfen, mit dest. Wasser und Aceton gespült und getrocknet. Dieses Material wird durch chemische Dampfabscheidung (CVD) mit 0,20* 10⁻⁴ g/cm² beschichtet. Hierzu wird Benzol bei 30°C in einem Blubberer durch einen Stickstofffluss über 30 Minuten in Kontakt mit der 1000°C heißen Metalloberfläche gebracht, bei den hohen Temperaturen zersetzt und als Film auf der Metalloberfläche abgeschieden. Die zuvor metallische Oberfläche wird schwarz glänzend nach der Abscheidung. Ein Test der Beschichtungshärte mit einem Bleistift, der in einem Winkel von 45° mit einem Gewicht von 1kg über die beschichtete Fläche gezogen wird ergibt bis zu einer Härte von 4B keine optisch wahrnehmbare Beschädigung der Oberfläche.
Ein Tesafilm Abziehtest, bei dem ein Tesa®-Streifen mit mindestens 3 cm Länge über 60 Sekunden mit dem Daumen auf die Oberfläche geklebt wird und anschließend im Winkel von 90° von der Oberfläche wieder abgezogen wird, ergibt deutlich sichtbare, graue Anhaftungen.

### Beispiel 7: Titan

Titan 99,6% als 0,1mm Folie (Goodfellow) wird einer 15 min. Ultraschallreinigung unterworfen, mit dest. Wasser und Aceton gespült und getrocknet. Dieses Material wird durch Tauchbeschichtung mit einem handelsüblichen Emballagelack mit 2,2*10⁻⁴ g/cm² beschichtet. Nach anschließender Pyrolyse mit Karbonisierung bei 800°C über 48 Stunden unter Stickstoff tritt ein Gewichtsverlust der Beschichtung auf 0,73*10⁻⁴ g/cm² ein. Die zuvor farblose Beschichtung wird matt, grau-schwarz glänzend. Ein Test der Beschichtungshärte mit einem Bleistift, der in einem Winkel von 45° mit einem Gewicht von 1kg über die beschichtete Fläche gezogen wird ergibt bis zu einer Härte von 8H keine optische Beschädigung der Oberfläche. Auch beispielweise mit einer Büroklammer kann die Beschichtung nicht zerkratzt werden. Ein Abziehtest, bei dem ein Tesa®-Streifen mit mindestens 3 cm Länge über 60 Sekunden mit dem Daumen auf die Oberfläche geklebt wird und anschließend im Winkel von 90° von der Oberfläche wieder abgezogen wird, ergibt keine Anhaftungen.

### Beispiel 8: Titan, veredelt mit CVD

Titan 99,6% als 0,1mm Folie (Goodfellow) wird einer 15 min. Ultraschallreinigung unterworfen, mit dest. Wasser und Aceton gespült und getrocknet. Dieses Material wird durch Tauchbeschichtung mit einem handelsüblichen Emballagelack mit 2,2*10⁻⁴ g/cm² beschichtet. Nach anschließender Pyrolyse mit Karbonisierung bei 800°C über 48 Stunden unter Stickstoff tritt ein Gewichtsverlust der Beschichtung auf 0,73*10⁻⁴ g/cm² ein. Dieses Material wird durch chemische Dampfabscheidung (CVD) weiter beschichtet mit 0,10*10⁻⁴ g/cm². Hierzu wird Benzol bei 30°C in einem Blubberer durch einen Stickstofffluss über 30 Minuten in Kontakt mit der 1000°C heißen, beschichteten Metalloberfläche gebracht, zersetzt und als Film auf der Oberfläche abgeschieden. Die zuvor metallische Oberfläche wird schwarz glänzend nach der Abscheidung. Nach der Abkühlung auf 400°C wird die Oberfläche durch Überleiten von Luft für die Dauer von 3 Stunden oxidiert. Ein Test der Beschichtungshärte mit einem Bleistift, der in einem Winkel von 45° mit einem Gewicht von 1kg über die beschichtete Fläche gezogen wird ergibt bis zu einer Härte von 8H keine optisch wahrnehmbare Beschädigung der Oberfläche. Ein Abziehtest, bei dem ein Tesa®-Klebebandstreifen mit mindestens 3 cm Länge über 60 Sekunde mit dem Daumen auf die Oberfläche geklebt wird und anschließend im Winkel von 90° von der Oberfläche wieder abgezogen wird, ergibt graue Anhaftungen.

## Patentansprüche

1. Verfahren zur Beschichtung von Substraten mit kohlenstoffbasiertem Material, umfassend die folgenden Schritte:
a) zumindest partielles Beschichten eines unter Karbonisierungsbedingungen temperaturbeständigen Substrates ausgewählt aus medizinischen Implantaten, Mikroelektronik- oder Mikromechanik-Bauteilen, Raschigringen, Sulzerpackungen, Kartuschen- und Filtersystemen, oder Isolierwerkstoffen, das Substrat ferner ausgewählt aus Metallen, Legierungen, Glas, Knochensubstanz oder Knochenimitate auf Kalziumkarbonatbasis, sowie Kombinationen davon, mit einem Polymerfilm an mindestens einer äußeren Oberfläche des Substrates, und anschließend
b) Karbonisierung des Polymerfilms in einer Inertgas-Atmosphäre bei Temperaturen im Bereich von 200°C bis 2500°C.

2. Verfahren nach Anspruch 1,
**dadurch gekennzeichnet, dass** der Polymerfilm Zusatzstoffe umfasst, ausgewählt aus der Gruppe der Füllstoffe, Porenbildner, Streckmittel, Schmiermittel, Pigmente, Siliziumoxide, Aluminiumoxide, Aluminosilikate, Zirkonoxide, Titanoxide, Talkum, Graphit; Ruß, Zeolith, Tonmaterialien, Phyllosilikate, Fullerene, Katalysatoren, Nanopartikel, anorganische Nanokomposite, Metalle, Metallsalze und Metallverbindungen.

3. Verfahren nach einem der vorhergehenden Ansprüche,
**dadurch gekennzeichnet, dass** der Polymerfilm Folien umfasst, ausgewählt aus Homo- oder Copolymeren von aliphatischen oder aromatischen Polyolefinen wie Polyethylen, Polypropylen, Polybuten, Polyisobuten, Polypenten; Polybutadien; Polyvinyle wie Polyvinylchlorid oder Polyvinylalkohol, Poly(meth)acrylsäure, Polyacrylnitril, Polyamid, Polyester, Polyurethan, Polystyrol, Polytetrafluorethylen, Wachse, Paraffinwachse, Fischer-Tropsch-Wachse, sowie Mischungen und Kombinationen dieser Homo- oder Copolymere.

4. Verfahren nach einem der Ansprüche 1 oder 2,
**dadurch gekennzeichnet, dass** der Polymerfilm ein Lackfilm ist, hergestellt aus einem Lack mit einer Bindemittelbasis aus Alkydharz, Chlorkautschuk, Epoxidharz, Acrylatharz, Phenolharz, Aminharz, Ölbasis, Nitrobasis, Polyester, Polyurethan, Teer, teerartige Materialien, Teerpech, Bitumen, Stärke, Zellulose, Schellack, Wachsen, organische Materialien aus nachwachsenden Rohstoffen, oder Kombinationen davon.

5. Verfahren nach einem der vorhergehenden Ansprüche,
**dadurch gekennzeichnet, dass** das kohlenstoffbasierte Material im Anschluss an die Karbonisierung einer oxidativen und/oder reduzierenden Nachbehandlung unterzogen wird.

6. Verfahren nach einem der vorhergehenden Ansprüche,
**dadurch gekennzeichnet, dass** das kohlenstoffbasierte Material im Anschluss an die Karbonisierung mittels Ionenimplantierung, oder einer CVD-Prozedur zur Abscheidung von Kohlenstoff und/oder Keramik modifiziert wird.

7. Verfahren nach einem der vorhergehenden Ansprüche,
**dadurch gekennzeichnet, dass** das Substrat aus medizinischen Implantaten ausgewählt aus Stents, chirurgisch-orthopädischen Knochenschrauben, Nägel oder Platten, Prothesen und Gelenksubstituten ausgewählt ist.

8. Verfahren nach einem der vorhergehenden Ansprüche,
**dadurch gekennzeichnet, dass** das beschichtete Substrat mit Wirkstoffen oder Mikroorganismen beladen wird.

9. Verfahren nach Anspruch 8,
**dadurch gekennzeichnet, dass** die aufgebrachten Wirkstoffe gezielt in einer Anwendungsumgebung freigesetzt werden können.

10. Verfahren nach einem der vorhergehenden Ansprüche,
**dadurch gekennzeichnet, dass** die Karbonisierung bei Temperaturen von 200° bis 1200 °C erfolgt.

11. Verfahren nach einem der vorhergehenden Ansprüche, umfassend
**dadurch gekennzeichnet, dass** die Karbonisierung in einer Inertgasatmosphäre ausgewählt aus Stickstoff und/oder Argon durchgeführt wird.

12. Verfahren nach einem der vorhergehenden Ansprüche,
**dadurch gekennzeichnet, dass** die resultierende kohlenstoffbasierte Beschichtung einen Kohlenstoffgehalt von mindestens 50 Gew.-%, vorzugsweise mindestens 60 Gew.-% und besonders bevorzugt mindestens 75 Gew.-% aufweist.

## Claims

1. A method for coating substrates with carbon-based material comprising the following steps:
a) at least partially coating of a substrate selected from medical implants, microelectronic- or micromechanic components, Raschig rings, Sulzer packings, cartridge systems and filter systems, and insulation materials, the substrate being temperature resistant at carbonization conditions and the substrate being further selected from metals, metal alloys, glass, bone or bone imitates based on calcium carbonate, as well as combinations thereof, with a polymer film on at least one of the outside surfaces of the substrate;
b) carbonizing the polymer film in an inert gas atmosphere at temperatures in the range of 200°C to 2500°C.

2. The method according to Claim 1,
**characterized in that** said polymer film comprises additives selected from the group of fillers, pore-forming agents, extenders, lubricants, pigments, silicon oxides, aluminum oxides, aluminum silicates, zirconium oxides, titanium oxides, talc, graphite, carbon black, zeolite, clay minerals, phyllosilicates, fullerenes, catalysts, nanoparticles, inorganic nanocomposites, metals, metal salts and metal compounds.

3. The method according to one of the preceding claims,
**characterized in that** said polymer film comprises films selected from homopolymers or copolymers of aliphatic or aromatic polyolefins such as polyethylene, polypropylene, polybutene, polyisobutene, polypentene, polybutadiene; polyvinyls such as polyvinyl chloride or polyvinyl alcohol, poly(meth)acrylic acid, polyacrylonitrile, polyamide, polyester, polyurethane, polystyrene, polytetrafluoroethylene, waxes, paraffin waxes, Fischer-Tropsch waxes, and mixtures and combinations of these homopolymers or copolymers.

4. The method according to any one of Claims 1 or 2,
**characterized in that** the polymer film is a lacquer film produced from a lacquer with a binder base of alkyd resin, chlorinated rubber, epoxy resin, acrylate resin, phenolic resin, amine resin, oil base, nitro base, polyester, polyurethane, tar, tar-like materials, tar pitch, bitumen, starch, cellulose, shellac, waxes, organic materials of renewable raw materials, or combinations thereof.

5. The method according to one of the preceding claims,
**characterized in that** the carbon-based material is subjected to an oxidative and/or reductive after-treatment following carbonization.

6. The method according to one of the preceding claims, **characterized in that** the carbon-based material is modified via ion implantation, or subjected to a CVD procedure for deposition of carbon and/or a ceramic, following carbonization.

7. The method according to one of the preceding claims,
**characterized in that** the substrate is selected from medical implants selected from stents, surgical or orthopedic bone screws, nails or plates, prostheses and joint substitutes.

8. The method according to one of the preceding claims,
**characterized in that** the coated substrate is loaded with active ingredients or microorganisms.

9. The method according to Claim 8,
**characterized in that** the applied active ingredients can be released in a controlled manner into an environment of use.

10. The method according to one of the preceding claims,
**characterized in that** the carbonization is performed at temperatures from 200 °C to 1200°C.

11. The method according to one of the preceding claims,
**characterized in that** the carbonization is conducted in an inert gas atmosphere selected from nitrogen and/or argon.

12. The method according to one of the preceding claims,
**characterized in that** the resulting carbon-based coating has a carbon content of at least 50 wt.-%, preferably at least 60 wt.-%, and most preferably at least 75 wt.-%.

## Revendications

1. Procédé de revêtement de substrats par du matériau à base de carbone, comprenant les étapes suivantes :
a) revêtement au moins partiel d'un substrat résistant à la température dans des conditions de carbonisation, sélectionné parmi des implants médicaux, des composants microélectroniques ou micromécaniques, des anneaux de Raschig, des garnissages du procédé Sulzer, des systèmes de cartouches et de filtres, ou des matériaux isolants, le substrat étant en outre sélectionné parmi des métaux, des alliages, du verre, de la substance osseuse ou des imitations d'os à base de carbonate de calcium, ainsi que des combinaisons de ces derniers, par un film polymère sur au moins une surface extérieure du substrat, puis
b) carbonisation du film polymère sous atmosphère de gaz inerte à des températures dans la plage de 200°C à 2500°C.

2. Procédé suivant la revendication 1, **caractérisé en ce que** le film polymère comprend des additifs, sélectionnés parmi le groupe des charges, agents porogènes, diluants, lubrifiants, pigments, oxydes de silicium, oxydes d'aluminium, aluminosilicates, oxydes de zirconium, oxydes de titane, talc, graphite, suie, zéolithe, matériaux argileux, phyllosilicates, fullerènes, catalyseurs, nanoparticules, nanocomposites inorganiques, métaux, sels métalliques et combinaisons métalliques.

3. Procédé suivant l'une des revendications précédentes, **caractérisé en ce que** le film polymère comprend des films, sélectionnés parmi des homo- ou copolymères de polyoléfines aliphatiques ou aromatiques, tels que polyéthylène, polypropylène, polybutylène, polyisobutylène, polypentène ; polybutadiène ; polyvinyles tels que chlorure de polyvinyle ou alcool polyvinylique, acide polyméthacrylique, polyacrylonitrile, polyamide, polyester, polyuréthanne, polystyrène, polytétrafluoroéthylène, cire, cire de paraffine, cire de Fischer - Tropsch, ainsi que des mélanges et des combinaisons de ces homo- ou copolymères.

4. Procédé suivant l'une des revendications 1 et 2,
**caractérisé en ce que** le film polymère est une pellicule de vernis, fabriquée à partir d'un vernis avec une base de liant en résine alkyde, caoutchouc chloré, résine époxy, résine d'acrylate, résine phénolique, résine amine, base d'huile, nitrobase, polyester, polyuréthanne, goudron, matériaux goudronneux, brai, bitume, amidon, cellulose, gomme-laque, cires, matières organiques de matières premières d'origine végétale, ou des combinaisons de ces derniers.

5. Procédé suivant l'une des revendications précédentes, **caractérisé en ce que** le matériau à base de carbone est soumis, à la suite de la carbonisation, à un post-traitement d'oxydation et/ou de réduction.

6. Procédé suivant l'une des revendications précédentes, **caractérisé en ce que** le matériau à base de carbone est modifié à la suite de la carbonisation au moyen d'une implantation ionique, ou d'un procédé CVD (dépôt chimique en phase vapeur) pour la séparation du carbone et/ou de la céramique.

7. Procédé suivant l'une des revendications précédentes, **caractérisé en ce que** le substrat est sélectionné parmi des implants médicaux, sélectionnés parmi des endoprothèses, des vis osseuses chirurgico-orthopédiques, des clous ou des plaques, des prothèses et des substituts d'articulations.

8. Procédé suivant l'une des revendications précédentes, **caractérisé en ce que** le substrat revêtu est chargé de substances actives ou de microorganismes.

9. Procédé suivant la revendication 8, **caractérisé en ce que** les substances actives appliquées peuvent être libérées sélectivement dans un milieu d'application.

10. Procédé suivant l'une des revendications précédentes, **caractérisé en ce que** la carbonisation s'effectue à des températures de 200° à 1200°C.

11. Procédé suivant l'une des revendications précédentes, **caractérisé en ce que** la carbonisation est réalisée sous atmosphère de gaz inerte sélectionnée parmi l'azote et/ou l'argon.

12. Procédé suivant l'une des revendications précédentes, **caractérisé en ce que** le revêtement résultant à base de carbone présente une teneur en carbone d'au moins 50% en poids, de préférence d'au moins 60% en poids, et de façon particulièrement préférentielle d'au moins 75% en poids.
